Europäisches Patentamt

(19) European Patent Office

Office européen des brevets

(11) EP 0 956 842 A1

(12) EUROPEAN PATENT APPLICATION

(43) Date of publication:
17.11.1999 Bulletin 1999/46

(51) Int. Cl.⁶: **A61F 13/15**, D06C 3/00, D06M 10/00, D06M 23/00, D04H 1/54

(21) Application number: 98108122.7

(22) Date of filing: 05.05.1998

(84) Designated Contracting States:
AT BE CH CY DE DK ES FI FR GB GR IE IT LI LU
MC NL PT SE
Designated Extension States:
AL LT LV MK RO SI

(71) Applicant:
THE PROCTER & GAMBLE COMPANY
Cincinnati, Ohio 45202 (US)

(72) Inventors:
• Fuchs, Christofer
61476 Kronberg (DE)

• Divo, Michael
61381 Friedrichsdorf (DE)
• Busam, Ludwig
65510 Hünstetten (DE)

(74) Representative:
Canonici, Jean-Jacques et al
Procter & Gamble European Service GmbH,
Sulzbacher Strasse 40-50
65824 Schwalbach am Taunus (DE)

(54) **Webs having deactivatable expansion obstruction means**

(57) The present invention relates to web materials which can be used in the production of large quantities of articles which are assembled from, inter alia, continuously fed web materials. In particular, the present invention teaches web materials which are expandable by a predetermined elongation after the expandability of the web material has been activated externally. To provide this functionality, the web materials comprise at least one longitudinal expansion means and at least one deactivatable expansion obstruction means. Another object of the present invention is the process for making such web materials comprising the step of incorporating longitudinal expansion means and deactivatable expansion obstruction means into the web material.

## Description

FIELD OF THE INVENTION

The present invention relates to web materials which can be used in the production of large quantities of articles which are assembled from, inter alia, continuously fed web materials. In particular, the present invention relates to those web materials which are expandable by a predetermined elongation after the user has activated the expandability of the web material.

BACKGROUND

Web materials are well known in the prior art, especially for use in the industrial manufacturing of large quantities of discrete articles. The web materials typically have a two dimensional configuration with the longitudinal dimension being substantially larger than the transverse dimension. Usually, the longitudinal dimension of a web material is also substantially larger than the length of the piece of web material actually used in the production of a single discrete article. During the manufacturing process, the web material is supplied in longitudinally continuous form and then cut into discrete pieces during the manufacturing process.

For many applications, it is preferable to use web materials which are longitudinally expandable without loosing their functionality. These web materials are especially useful when they are attached to elements of varying size or position. The expandability of the web material allows them to adapt to the new size or position of the element they are attached to.

In some cases, it is desirable that the expansion of the web material is limited to a certain elongation. Such a need arises when the web material is used to guide or to limit the growth or movement of the attached element as described above.

It can also be advantageous when the time of the expansion of the web material can be determined externally. This specific need exists, for example, when the time of growth or movement of the attached element should be controlled.

In U.S. patent applications No. 5,518.801 issued to Chappell, No. 5,650,214 issued to Anderson, and No. 5,691,035 issued to Chappell, web materials exhibiting elastic-like behavior are disclosed. Specifically, these web materials are described to have an elongation and recovery with a definite and sudden increase in the force resisting elongation where this definite and sudden increase in resistive force restricts further elongation against relatively small elongation forces.

Whilst progress has been made towards a web material that is expandable by a predetermined amount, this expansion can only be started by increasing the tension on the web material.

There remains, however, the problem to provide a web material that can be easily expanded by a predetermined amount after the expandability has been externally activated during use.

The present invention is a web material comprising a longitudinal dimension, a transverse dimension substantially smaller than the longitudinal dimension, at least one longitudinal expansion means, and at least one deactivatable expansion obstruction means.

It is a further object of the present invention to provide such a web material that additionally has a Relative Elastic Modulus Reduction of at least 50%, preferably at least 70%, even more preferably at least 90%, when it is submitted to the Deactivated Expansion Obstruction Test.

It is a further object of the present invention to provide such a web material that comprises a plurality of longitudinal expansion means longitudinally spaced apart at fixed distances.

It is a further object of the present invention to provide such a web material additionally having a plurality of longitudinal expansion means, the minimum distance between the juxtaposed transversely delimiting edges of two adjacent longitudinal expansion means being D1 and the maximum distance between the two transversely delimiting edges of a longitudinal expansion means being D2 wherein the ratio of D1 to D2 is at least two preferably at least three.

It is a further object of the present invention to provide such a web material which comprises a region having a Relative Expandability After Deactivation of at least 50%, preferably at least 100%, more preferably at least 150% according to the Expansion After Deactivation Test.

It is a further object of the present invention to provide such a web material in which the deactivatable expansion obstruction means are deactivatable by a cutting device, by being cut into at least two parts, by being cut out of the web material, by electromagnetic radiation, by adding a deactivation agent, or by applying a longitudinal web tension.

It is a further object of the present invention to provide such a web material in which the deactivatable expansion obstruction means are bonds between regions of the web material which are spaced apart longitudinally along the surface contour. Such bonds may be thermobonds, adhesive bonds, mechanical fixation, fiber entanglement. The configuration of the bonds may be continuous or non-continuous in transverse direction. The bonds may be positioned close to the longitudinally extending edges of the web material

It is a further object of the present invention to provide such a web material comprising a first surface, a second surface opposite the first surface, a first hidden surface, and a second hidden surface opposite the second hidden surface wherein the longitudinal expansion means comprises at least one pair of a first transverse fold and a second transverse fold, the first transverse fold being secured by the deactivatable expansion obstruction means such that at least part of the first hidden surface between the first transverse folding edge and the second transverse folding edge is in contact with a part of the first hidden surface on the opposite side of the first transverse folding edge, the second transverse fold being secured by the deactivatable expansion obstruction means such that at least part of the second hidden surface between the first transverse folding edge and the second transverse folding edge is in contact with a part of the second hidden surface on the opposite side of the second transverse folding edge.

It is a further object of the present invention to provide such a web material in which the longitudinal expansion means comprises a plurality of pairs of a secured first transverse fold and a secured second transverse fold.

It is a further object of the present invention to provide such a web material in which the longitudinal expansion means comprises a transversely corrugated region of the web material.

It is a further object of the present invention to provide such a web material according to Claim comprising a first region and a second region wherein the first region has a higher basis weight than the second region.

It is a further object of the present invention to provide such a web material that has a Basis Weight Deviation of less than 5% when submitted to the Basis Weight Deviation Test.

It is an additional object of the present invention to provide a process for making a web material comprising the steps of forming a web, stabilizing a web, incorporating longitudinal expansion means into a web, and incorporating deactivatable expansion obstruction means into a web.

It is a further object of the present invention to provide such a process for making a web material wherein the steps of forming and stabilizing a web precede the step of incorporating longitudinal expansion means and deactivatable expansion obstruction means into the web.

It is a further object of the present invention to provide such a process for making a web material wherein the step of incorporating longitudinal expansion means and deactivatable expansion obstruction means into a web is intermediate the step of forming a web and the step of stabilizing a web.

It is a further object of the present invention to provide such a process for making a web material according to Claim wherein the step of incorporating longitudinal expansion means into the web material comprises at least one step selected from the group of: transverse folding a region of the web material, z-folding a region the web material, accordion folding the web material.

It is a further object of the present invention to provide such a process for making a web material wherein the step of incorporating longitudinal expansion means into the web material comprises at least one step selected from the group of: creping a region of the web material, corrugating a region of the web material, ring rolling a region of the web material, or pleating a region of the web material.

It is a further object of the present invention to provide such a process for making a web material wherein the step of incorporating deactivatable expansion obstruction means into the web material uses a bonding mechanism of the adhesive bonding type.

It is a further object of the present invention to provide such a process for making a web material wherein the step of incorporating deactivatable expansion obstruction means into the web material uses a bonding mechanism selected from the group of: ultrasonic bonding, heat bonding, pressure bonding, friction bonding, or autogenous bonding

It is a further object of the present invention to provide such a process for making a web material wherein the step of incorporating deactivatable expansion obstruction means into the web material uses the bonding mechanism of mechanically fixing.

It is a further object of the present invention to provide such a process for making a web material further comprising the steps of: unwinding the web, longitudinally slitting the web, and rewinding the web, which is characterized in that the step of incorporating the longitudinal expansion means and the deactivatable expansion obstruction means into the web material precedes the step of unwinding, is intermediate the step of unwinding and the step of rewinding, or follows the step of rewinding.

DETAILED DESCRIPTION OF THE INVENTION

The present invention relates to web materials which are used in the production of large quantities of articles which are assembled from, inter alia, continuously fed web materials. Preferably, these web materials are supplied as roll stock.

The term "web material" as used herein refers to a sheet-like material, or to a composite or laminate comprising two or more sheet-like materials. For example, a web material can be a fibrous web, a non-fibrous web, a foam, or the like.

The web material of the present invention is essentially two-dimensional, i.e. the thickness of the web material is much smaller than its longitudinal and its transverse dimension. Additionally, the transverse dimension of the web material is substantially smaller than its longitudinal dimension. The longitudinal dimension preferably exceeds the transverse dimension by a factor of 100, most preferably the longitudinal dimension of the web material of the present invention is essentially infinite.

Furthermore, the web material of the present invention has a first external surface and a second external surface opposite the first surface. The web material of the present invention may also comprise hidden surfaces including first and second hidden surfaces according to which external surface they are connected to. At least part of each hidden surface is in contact with at least a part of one other hidden surface such as after folding a conventional web material. These hidden surfaces may become part of the respective external surface during the expansion of the web material of the present invention.

One embodiment of the web material of the current invention is a fibrous web, such as a tissue web, a non-woven web, a woven web, a knit web, or the like. Such fibrous webs can be made from natural fibers (e.g., wood or cotton fibers), synthetic fibers (e.g., polyester or polypropylene fibers), or a combination of natural and synthetic fibers. The non-woven web materials may be - without limiting to these - made by processes commonly referred as spunlace, spunbond, meltblown, carded, and/or air-through or calendar bonded. The fibrous webs of the present invention may be absorbent or non-absorbent, liquid pervious, or liquid impervious.

Another embodiment of the web material of the present invention is non-fibrous web such as a film. Non-fibrous web materials of the present invention may be comprised of polyolefins such as polyethylenes, including linear low density polyethylene (LLDPE), low density polyethylene (LDPE), ultra low density polyethylene (ULDPE), high density polyethylene (HDPE), or polypropylene and blends thereof with the above and other materials. Examples of other suitable polymeric materials which may also be used include, but are not limited to, polyester, polyurethanes, compostable or biodegradable polymers, heat shrink polymers, thermoplastic elastomers, metallocene catalyst-based polymers (e.g., INSITE™. available from Dow Chemical Company and Exxact™ available from Exxon), and breathable polymers.

The non-fibrous web material may also be comprised of an apertured film, macroscopically expanded three-dimensional formed film, absorbent or foam, filled composition, or laminates and/or combinations thereof.

Web materials of the present invention may include laminates of the above mentioned materials. Laminates may be combined by any number of bonding methods known to those skilled in the art. Such bonding methods include but are not limited to thermal bonding, adhesive bonding (using any of the number of adhesives including but not limited to spray adhesives, hot melt adhesives, latex based adhesives and the like), sonic bonding and extrusion laminating whereby a polymeric film is cast directly onto a substrate, and while still in a partially molten state, bonds to one side of the substrate, or by depositing meltblown fibers non-woven directly onto a substrate.

Alternatively, the web material of the present invention may also comprise discretely distributed substances which are attached to the web material.

An essential element of the web material of the present invention is that it comprises at least one longitudinal expansion means.

The term "longitudinal expansion means" as used herein refers to a means that allows the web material to expand in longitudinal direction by a predetermined amount. After this expansion, the web material preferably exhibits a behavior under longitudinal tension similar to a conventional web material.

Preferably, the longitudinal expansion of a web material of the present invention is irreversible, after the predetermined longitudinal expansion there is no contraction force pulling the web material back to its unexpanded configuration.

Generally, a region of the web material whose perimeter coincides with the perimeter of a longitudinal expansion means can be characterized by having an at least partially curved longitudinal surface contour. As a result, the longitudinal surface contour length of this web material region is substantially longer than the longitudinal distance of the two transverse edges delimiting the region. Specifically, the difference between the surface contour length and the longitudinal distance between the delimiting transverse edges is the predetermined expansion length accessible to the region of the web material comprising the longitudinal expansion means.

The term "longitudinal surface contour length" as used herein refers to the length of a region of a web material whereby the length is measured along a possibly curved path which follows the longitudinal dimension of the external and hidden surfaces as they are connected to each other.

A preferred embodiment of the longitudinal expansion means of the present invention is a double transverse fold, which upon unfolding allows the web material to substantially increase its longitudinal dimension.

A particularly preferred embodiment of the longitudinal expansion means of the present invention having a curved longitudinal surface contour is comprised in a web material according to the present invention which is obtained by arranging a conventional precursor web material in a transverse z-fold.

The term "z-fold" as used herein refers to two transverse folds which are arranged such that the longitudinal cross section of the web material looks like the letter "z" when looked at from the side. Specifically, the first surface of the precursor web material between the first transverse fold and the second transverse fold is in close proximity to the first surface on the opposite side of the first transverse fold and the second surface of the precursor web material between the first transverse fold and the second transverse fold is in close proximity to the second surface on the opposite side of the second transverse fold. The z-fold allows the web to comprise a longitudinal expansion means without loosing its primarily two dimensional configuration.

Another preferred embodiment of the longitudinal expansion means of the present invention is a plurality of transverse folds which are in close proximity to each other, called accordion fold hereafter.

The longitudinal expansion means of the present invention may also be but are not limited to regions of the web material which are mechanically strained, creped, corrugated, "ring rolled", or pleated. The process of "ring rolling" is described in U. S: patent No. 4,517,714 issued to Sneed. All of these treatments have to be carried out in the transverse direction to render the web material longitudinally expandable.

A particularly preferred embodiment of the web material of the present invention comprises a plurality of longitudinal expansion means which are spaced apart longitudinally allowing the web material to locally expand at the positions of the longitudinal expansion means. An even more preferred embodiment comprises longitudinal expansion means at longitudinally equal distances to allow a periodic local expansion of the web material when it is converted.

Another essential element of the web material of the present invention that it comprise vs. at least one deactivatable expansion obstruction means.

The term "deactivatable expansion obstruction means" as used herein refers to a means that is preventing the expansion of a web material comprising a longitudinal expansion means. Furthermore, the deactivatable expansion obstruction means is designed such that it can be deactivated by an external deactivation device. After deactivation of the deactivatable expansion obstruction means the longitudinal expansion means can be utilized to longitudinally expand the web material at the position of the respective longitudinal expansion means.

The term "external deactivation device" as used herein refers to any apparatus that is capable of directly or indirectly acting on the web material thereby removing at least one deactivatable expansion obstruction means or rendering at least one deactivatable expansion obstruction means non-functional.

Generally, the deactivatable expansion obstruction means of the present invention holds the delimiting transverse edges of a web material region comprising a longitudinal expansion means at a distance smaller than the longitudinal surface contour length between the two delimiting transverse edges.

Preferably, the tearable expansion obstruction means impedes the relative motion of hidden surface regions of the web material which are spaced apart along the longitudinal surface contour. This can be achieved by at least partial direct bonding or at least partial indirect bonding such as edge bonding.

The deactivatable expansion obstruction means of the present invention include but are not limited to adhesive bonding, ultrasonic bonding, heat bonding, pressure bonding, friction bonding, autogenous bonding or combinations of different bonding methods.

The deactivatable expansion obstruction means may also be a mechanical fixation by a mechanical fixation device such as a bracket, a thread or by fiber entanglement.

Alternatively, the tearable expansion obstruction means may be region of the web material with low or zero longitudinal expandability which is arranged longitudinally parallel to a longitudinally expandable region of the web material.

An embodiment of the external deactivation device may be but is not limited to a cutting device, a electromagnetic radiation emitting device, a heating device, a web tensioning device, a deactivation agent dispensing device, or the like.

A preferred embodiment of the deactivatable expansion obstruction means can be deactivated by electromagnetic radiation (infrared, visible, ultraviolet), or a deactivation agent (water, organic solvent, chemical agent). An even more preferred embodiment of the deactivatable expansion obstruction means is an adhesive bond that can be deactivated by electromagnetic radiation or by a deactivation agent.

In another preferred embodiment of the web material of the present invention, the deactivatable expansion obstruction means are positioned in proximity to the longitudinal edges of the web material. Alternatively, the deactivatable expansion obstruction means can be arranged in isolated spaced apart positions. Preferably, deactivation of the deactivatable expansion obstruction means is achieved by cutting the deactivatable expansion obstruction means out of the web. Alternatively, deactivation of the deactivatable expansion obstruction means is accomplished by cutting the deactivatable expansion obstruction means in at least two parts.

In yet another preferred embodiment of the web material of the present invention, the deactivatable expansion obstruction means can be torn apart by applying a longitudinal tension to the web material without tearing the entire web. In this case the tear force of the deactivatable expansion obstruction means must be substantially lower than the tear force of the web material.

In yet another embodiment of the present invention, the mechanical fixation can be removed or rendered non-functional by an external deactivation device such as a cutting device.

The Deactivated Expansion Obstruction Test disclosed in the present invention quantifies the ability of a web material to more easily expand after deactivation of its deactivatable expansion obstruction means. The parameter describing this ability is the relative Elastic Modulus Reduction exhibited while measuring two identical samples, one of them comprising deactivated expansion obstruction means.

When the web material of the present invention is submitted to the Deactivated Expansion Obstruction Test the Elastic Modulus Reduction preferably is at least 50%, more preferably at least 75%, even more preferably at least 90%.

A preferred embodiment of the present invention is a web material that comprises a first region and a second region where the second region has a higher basis weight than the first region. In an even more preferred embodiment of the web material of the present invention, the second region comprises at least one longitudinal expansion means whereas the first region does not comprise a longitudinal expansion means. Even more preferably, the basis weight of the second region is chosen such that after the expansion by means of the longitudinal expansion means the basis weight of the second region is essentially similar to the basis weight of the first region. This property is measured using the Basis Weight Deviation Test. Preferably, the Relative Basis Weight Deviation of the web material of the present invention is less than 10%, more preferably less than 5%. The advantage of this particular embodiment is that after the expansion by the predetermined amount the web material has an essentially uniform basis weight.

Preferably, the web material of the present invention or at least a region thereof which is comprising at least one longitudinal expansion means has a Relative Expandability after Deactivation of at least 50%, more preferably of at least 100%, even more preferably of at least 150%. This parameter describes the relative expandability the web material of present invention exhibits after deactivation of the expansion obstruction means.

The Relative Expandability after Deactivation is quantified with the Expansion After Deactivation Test disclosed in this application.

Another aspect of the present invention is the process for making a web material according to the present invention. Alternatively, the web material of the present invention can also be obtained by modifying a conventional web material.

Preferably, the process of the present invention of making a web material comprises the steps of (A) forming a web material, (B) stabilizing the web material, (C) incorporating unexpanded longitudinal expansion means into the web material, (D) incorporating deactivatable expansion obstruction means into the precursor web material, and optionally, (E) unwinding the web material, optionally, (F) longitudinally slitting the web material optionally, (G) rewinding the web material.

Therein, the combination of incorporating longitudinal expansion means into a conventional precursor web with the incorporation of deactivatable expansion obstruction means in order to prevent the expansion of the longitudinal expansion means allows the production of web materials according to the present invention.

The order of the steps does not necessarily have to be in the above order. It is possible to carry out step B at any point after step A, in particular after step D. Steps C and D may also be carried out intermediate steps E and G or after step G.

Preferably, the step of incorporating longitudinal expansion means into the web material is carried out by transversely folding the web, even more preferably in a z-fold or in an accordion-fold. Alternatively, the longitudinal expansion means are incorporated into the web by at least partially prestretching the web material to render it longitudinally expandable. Possible processes for this task are creping, corrugating, "ring rolling", or pleating. All of these treatments have to be carried out in the transverse direction to render the web material longitudinally expandable.

Preferably, the deactivatable expansion obstruction means are incorporated into the web material by bonding surfaces or edges which spaced apart along the surface contour. These surfaces or edges have been brought into close proximity to each other by the incorporation of the longitudinal expansion means into the web material. Possible methods to achieve these bonds include but are not limited to adhesive bonding, ultrasonic bonding, heat bonding, pressure bonding, friction bonding, autogenous bonding or combinations of different bonding methods. Alternatively, the bonds can be achieved by incorporating mechanical fixation devices such as brackets, threads, or the like into the web material. Another possibility to incorporate deactivatable expansion obstruction means into the web material is the entanglement of fibers of different surface or edge regions e.g. by needling, hydroentangling, or the like.

Z-folded non-woven web material

This example is provided to demonstrate the principle of the present invention.

A spunbond-meltblown-spunbond non-woven web material, available from Corovin GmbH of Peine, Germany, under the designation MD3000, mostly consisting of polypropylene fibers was cut in a longitudinal strip having a length of 20 centimeters and a width 2.54 centimeters. The web material was arranged in a transverse z-fold by the following steps:

At positions located 5 and 8 centimeters away from one of the transverse edges, the strip of web material was transversely folded.

The transverse fold located at 8 centimeters was folded back onto the web material touching it about two centimeters away from the transverse edge.

To secure the z-fold the longitudinal edges of the three layers forming the z-fold were heat bonded up to a depth of one millimeter by applying a temperature a temperature slightly higher than the melting point of the fibers of the non-woven web material.

The final length of the z-folded strip of web material was 140 millimeters.

The Elastic Modulus Reduction of Example 1 was 99 % when it was submitted to Deactivated Expansion Obstruction Test.

METHODS

Elastic Modulus Test

The Elastic Modulus Test is used for measuring the elastic modulus which is defined as the slope of the expansion tension vs. relative elongation curve at 0% relative elongation. The tests are performed on an standard stress-strain analysis apparatus such as a Zwick Model 1445, available from Zwick GmbH & Co. of Ulm, Germany, which is interfaced to a Compaq Prolinea 466 computer available from Compaq Computer Corporation of Houston/Texas, USA, using Zwick 7047.4b software which is available from Zwick GmbH & Co. of Ulm, Germany. All essential parameters needed for testing are input in the software for each test. Also, all data collection, data analysis and graphing are done using the software.

The samples used for this test are 25.4 millimeters wide by 140 millimeters long with the long axis of the sample cut parallel to the longitudinal dimension of the web material. The sample should be cut with a sharp die cutter or some suitably sharp cutting device designed to cut a (25.4+/-1) millimeter wide sample. The sample should be cut so that an area representative of the longitudinal expansion means is represented. There will be cases (due to variations in either the size or distance of the longitudinal expansion means) in which it will be necessary to cut either larger or smaller samples than is suggested herein. In this case, it is very important to note (along with any data reported) the size of the sample, which area of the web material it was taken from, and preferably include a schematic diagram of the representative area used for the sample. Also, the results need to be calculated taking into account the different length. Three samples of a given material are tested.

The grips of the apparatus consist of air actuated grips designed to concentrate the entire gripping force along a single line perpendicular to the direction of testing stress having one flat surface and an opposing face from which protrudes a half round to minimize slippage of the sample. The distance between the lines of gripping force should be 100 millimeters as measured by a steel rule held beside the grips. This distance will be referred to from hereon as the "gauge length". The sample is mounted in the grips with its long axis perpendicular to the direction of applied percent elongation. The crosshead speed is set to 500 millimeter per minute. The crosshead elongates the sample until the sample breaks at which point the crosshead stops and returns to its original position (0% elongation).

The elastic modulus measured by the apparatus for the three samples are averaged to obtain the final result.

Deactivatable Expansion Obstruction Test

The Deactivatable Expansion Obstruction Test is used to measure the Relative Elastic Modulus Reduction of a web material.

First, six identical samples of the web material, called A1, A2, A3, B1, B2, and B3 hereafter, are prepared following the instructions given in the Elastic Modulus Test. Each sample should comprise at least one longitudinal expansion means and at least one deactivatable expansion obstruction means. In case the sample size of the Elastic Modulus Test is sufficient to fulfill this requirement, the Elastic Modulus Test has to modified to accommodate sufficiently large web material samples.

The samples A1, A2, and A3 are submitted to the Elastic Modulus Test. The resulting elastic moduli are averaged to obtain the elastic modulus EMA of the samples A1, A2, and A3. Then, the deactivatable expansion obstruction means comprised in the samples B1, B2, and B3 are deactivated and the modified samples are submitted to the Elastic Modulus Test. The resulting elastic moduli are averaged to obtain the elastic modulus EMB of the samples B1, B2, and B3.

Finally, the Relative Elastic Modulus Reduction is computed according to the formula (EMA - EMB) / EMA .

Basis Weight Deviation Test

This test is used to determine the uniformity of a web material after the deactivatable expansion obstruction means comprised in the web material have been deactivated.

Three identical samples of the submitted web material, named samples A1, A2, and A3 hereafter, are prepared according to the sample preparation described in the Elastic Modulus Test. Each sample should comprise at least one longitudinal expansion means and at least one deactivatable expansion obstruction means. In case the sample size of the Elastic Modulus Test is insufficient to fulfill this requirement, the Elastic Modulus Test has to be modified to accommodate sufficiently large web material samples.

Sample A1 is mounted in the grips of a standard stress-strain measurement apparatus such as a Zwick Model 1445, available from the Zwick GmbH & Co. of Ulm, Germany, according to the instructions of the Elastic Modulus Test. Subsequently, sample A1 is expanded with an expansion tension of 1 Newton per 0.0254 meter using the apparatus. Finally, five square pieces of web material having a surface area of (1+/- 0.01) square centimeter are cut from sample A1. The positions at which the pieces are cut out should be chosen equally distributed along the longitudinal dimension of sample A1 and should be centered with regard to the transverse direction. All pieces of web material obtained in this fashion are weighed with a precision of one microgram. The same procedure is carried out with samples A2 and A3.

The Relative Basis Weight Deviation is obtained by dividing the standard deviation of the weights of the 15 pieces of web material by the average weight of the pieces.

Expansion After Deactivation Test

The Expansion After Deactivation Test is used to measure the relative expandability of a web material after the deactivatable expansion obstruction means comprised in the web material are deactivated.

First, three identical samples of the web material, called B1, B2, and B3 hereafter, are prepared following the instructions given in the Elastic Modulus Test. Each sample should comprise at least one longitudinal expansion means and at least one deactivatable expansion obstruction means. In case the sample size of the Elastic Modulus Test is sufficient to fulfill this requirement, the Elastic Modulus Test has to modified to accommodate sufficiently large web material samples.

The deactivatable expansion obstruction means of the samples B1, B2, and B3 are deactivated. Afterwards, the same procedure as for sample A1 is carried out for samples B1, B2, and B3 to measure the relative elongation under an expansion tension of 1 Newton per 0.0254 meter. The resulting relative elongation are averaged obtaining the Relative Expandability After Deactivation.

While the test methods described above are useful for many of the web materials of the present invention it is recognized that the test method may have to be modified to accommodate some of the more complex web materials within the scope of the present invention.

**Claims**

1. An web material having

   - a longitudinal dimension, and
   - a transverse dimension substantially smaller than said longitudinal dimension
   - and comprising at least one longitudinal expansion means characterized in that said web material further comprises at least one deactivatable expansion obstruction means.

2. A web material according to Claim 1, wherein said web material has an Relative Elastic Modulus Reduction of at least 50% when submitted to the Deactivated Expansion Obstruction Test.

3. A web material according to Claim 1, wherein said deactivatable expansion obstruction means is located in a region having a Relative Expandability After Deactivation of at least 50% when submitted to the Expansion After Deactivation Test.

4. A web material according to Claim 1, wherein said web material comprises a plurality of said longitudinal expansion means spaced apart at fixed distances.

5. A web material according to Claim 4, the minimum longitudinal distance between the juxtaposed transversely delimiting edges of two adjacent longitudinal expansion means being D1, and the maximum longitudinal distance between the two transversely delimiting edges of a longitudinal expansion means being D2 wherein the ratio of D1 to D2 is at least two.

6. A web material according to Claim 1, wherein said deactivatable expansion obstruction means are bonds between regions of said web material which are spaced apart longitudinally along the surface contour.

7. A web material according to Claim 6, wherein said bonds are continuous in transverse direction.

8. A web material according to Claim 6, wherein said bonds are non-continuous in transverse direction.

9. A web material according to Claim 1, comprising a first surface and a second surface wherein said longitudinal expansion means comprises at least one pair of a first transverse fold and a second transverse fold, said first transverse fold being secured by said deactivatable expansion obstruction means such that at least part of said first hidden surface between said first transverse folding edge and said second transverse folding edge is in contact with a part of said first hidden surface on the opposite side of said first transverse folding edge, said second transverse fold being secured by said deactivatable expansion obstruction means such that at least part of said second hidden surface between said first transverse folding edge and said second transverse folding edge is in contact with a part of said second hidden surface on the opposite side of said second transverse folding edge.

10. A web material according to Claim 1, comprising a first region and a second region wherein said first region has a higher basis weight than said second region.

11. A process for making a web material comprising the steps of

- forming a web
- stabilizing said web
- incorporating longitudinal expansion means into said web
- incorporating deactivatable expansion obstruction means into said web.

12. A process for making a web material according to Claim 11, wherein said steps of forming and stabilizing a web precede said step of incorporating longitudinal expansion means and deactivatable expansion obstruction means into the web.

13. A process for making a web material according to claim 11, wherein said step of incorporating longitudinal expansion means and deactivatable expansion obstruction means into a web is intermediate said step of forming a web and said step of stabilizing a web.

14. A process for making a web material according to Claim 11, wherein said step of incorporating longitudinal expansion means into the web material comprises at least one step selected from the group of:

- transverse folding a region of said web material
- z-folding a region said web material
- accordion folding said web material

15. A process for making a web material according to Claim 11, further comprising the steps of - unwinding said web - longitudinally slitting said web - rewinding said web characterized in that said step of incorporating said longitudinal expansion means and said deactivatable expansion obstruction means into said web material precedes said step of unwinding said web.

## EUROPEAN SEARCH REPORT

**European Patent Office**

**Application Number**

EP 98 10 8122

### DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | EP 0 650 714 A (PROCTER & GAMBLE) 3 May 1995 * column 6, line 9 - line 13; claims; figures * | 1,4,11, 12 | A61F13/15 D06C3/00 D06M10/00 D06M23/00 D04H1/54 |
| X | EP 0 266 158 A (ROBINSON & SONS LTD) 4 May 1988 | 1,4,11, 12 | |
| A | * the whole document * | | |
| X | EP 0 689 816 A (KIMBERLY CLARK CO) 3 January 1996 * claims * | 1-3,11, 12 | |
| X | US 5 259 902 A (MUCKENFUHS DELMAR R) 9 November 1993 * column 1, line 55 - column 2, line 12; claims; figures * | 1,4 | |
| A | * column 5, line 59 - line 68 * | 2,3,5, 11,12 | |
| | * column 11, line 27 - line 35 * | | |
| X | DE 28 10 680 A (HARTMANN PAUL AG) 20 September 1979 * claims; figures * | 1,4 | TECHNICAL FIELDS SEARCHED (Int.Cl.6) D06C D06M |
| A | | 11 | D04H A61F |
| X | US 5 468 428 A (HANSCHEN THOMAS P ET AL) 21 November 1995 * column 18, line 21 - line 28; claims; examples; tables * | 1,4 | A41B B29C |
| A | | 11 | |
| X | US 3 694 815 A (BURGER WILLIAM H) 3 October 1972 * the whole document * | 1 | |
| A | | 2,3,11 | |
| | -/-- | | |

The present search report has been drawn up for all claims

| Place of search | Date of completion of the search | Examiner |
|---|---|---|
| THE HAGUE | 28 September 1998 | Mirza, A |

European Patent
Office

# EUROPEAN SEARCH REPORT

Application Number

EP 98 10 8122

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | EP 0 572 116 A (KATO TALL ;ZIN CO LTD (JP)) 1 December 1993<br>* abstract; figures * | 1 | |

TECHNICAL FIELDS SEARCHED (Int.Cl.6)

The present search report has been drawn up for all claims

| Place of search | Date of completion of the search | Examiner |
|---|---|---|
| THE HAGUE | 28 September 1998 | Mirza, A |

EPO FORM 1503 03.82 (P04C01)